# EUROPÄISCHE PATENTSCHRIFT

(11) **EP 2 770 942 B1**
(45) Veröffentlichungstag und Bekanntmachung des Hinweises auf die Patenterteilung: **12.02.2020**
(21) Anmeldenummer: 12766595.8
(22) Anmeldetag: 27.09.2012
(51) Int. Cl.: A61C 8/00

(54) **LABORIMPLANTAT MIT INDEXIERUNGEN ZUM EINBRINGEN IN KUNSTSTOFFMODELLE MIT ENTSPRECHENDEN GEGENINDEXIERUNGEN**
LABORATORY IMPLANT WITH INDEXING FOR INSERTION INTO PLASTIC MODELS WITH CORRESPONDING COUNTER-INDEXING
IMPLANT DE LABORATOIRE AVEC INDEXATIONS DESTINÉ À L'INCORPORATION DANS DES MODÈLES EN MATIÈRE PLASTIQUE PRÉSENTANT DES INDEXATIONS CONJUGUÉES CORRESPONDANTES

(30) Priorität: 07.10.2011 DE 102011115031
(43) Veröffentlichungstag der Anmeldung: 03.09.2014
(73) Patentinhaber: Kulzer GmbH, 63450 Hanau (DE)
(72) Erfinder: UEBERÜCK, Norbert, 63477 Maintal (DE)
(74) Vertreter: Bendele, Tanja
(86) Internationale Anmeldenummer: PCT/EP2012/004043
(87) Internationale Veröffentlichungsnummer: WO 2013/050116

(56) Entgegenhaltungen:
- EP-A1- 1 704 830
- WO-A1-2010/115443
- WO-A2-2007/005490
- WO-A2-2012/095851
- DE-U1-202011 001 969

## Beschreibung

Die Erfindung betrifft ein Verfahren zur Herstellung eines dreidimensionalen Modells zumindest eines Teilbereichs eines Kiefers zum Herstellen eines Zahnersatzes, bei dem ein Laborimplantat in einer Ausnehmung des Modells angeordnet wird.

Die Erfindung betrifft auch ein Modell eines Kiefers zur Herstellung eines Zahnersatzes, insbesondere zum Durchführen eines solchen Verfahrens umfassend eine Ausnehmung zum Einsetzen eines Laborimplantats; sowie ein Laborimplantat zur Herstellung eines Zahnersatzes, insbesondere zum Durchführen eines solchen Verfahrens, umfassend einen Bereich zum Einstecken in eine Ausnehmung eines Modells eines Kiefers und einen Kopfteil zum Aufbau des Zahnersatzes.

Dentalimplantate werden in der Zahnmedizin eingesetzt, um extrahierte oder ausgefallene Zähne zu ersetzen. Dazu werden die Dentalimplantate in den Kieferknochen eingesetzt. Aus der DE 10 2007 029 105 A1 sind Dentalimplantate bekannt, die einen Pfostenteil umfassen, der in den Kieferknochen eingesetzt wird, und einen Aufbauteil, auf dem ein Zahnersatz aufgebracht wird. Der Pfostenteil und der Aufbauteil werden mit einer Schraube verbunden. Zudem sind der Pfostenteil und der Aufbauteil mit Indizierungen strukturiert, die verhindern sollen, dass der Aufbauteil in beliebigen Winkeln in den Pfostenteil eingesetzt werden kann.

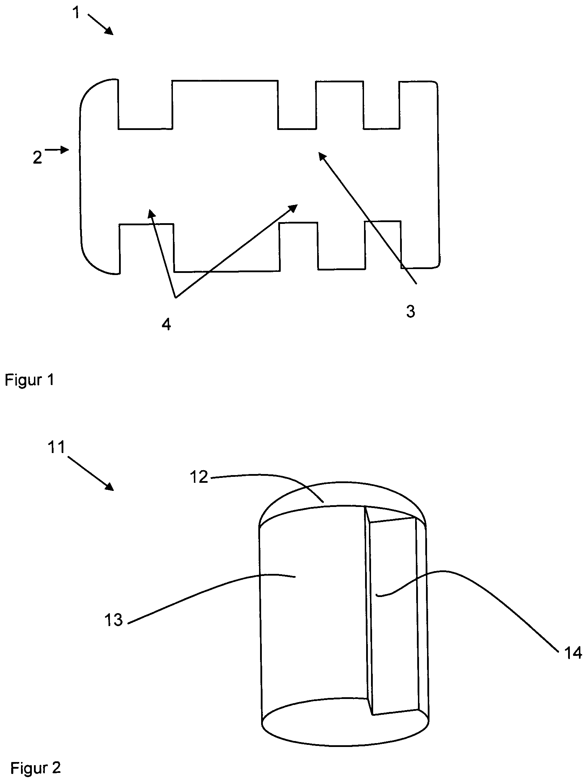

Laborimplantate werde dazu benötigt, einem Zahntechniker die Möglichkeit zu geben, einen Zahnersatz im Labor zu modellieren, der dann später beim Patienten eingesetzt wird. Laborimplantate haben dazu eine materialspezifische Geometrie und Form. Meist wird dazu ein Abdruck mit einer Abformmasse genommen, mit dem dann anschließend ein Gipsmodell des Kiefers des Patienten gefertigt wird, indem der Abdruck mit Gips ausgegossen wird. Das Laborimplantat wird dann in das Modell des Kiefers in der entsprechenden Position eingesetzt. Die Geometrie und Form des Laborimplantats muss dazu auf den Gips angepasst werden. Dazu sind teilweise Unterschnitte vorgesehen, in welche der flüssige Gips einfließen kann und der nach dem Aushärten das Laborimplantat in der entsprechenden Position und Lage hält. Ein solches Laborimplantat nach dem Stand der Technik ist in Figur 1 in schematischer Querschnittansicht dargestellt. Dieses Modell des Kiefers wird mit dem integrierten Laborimplantat vom Zahntechniker dazu verwendet, den Zahnersatz passend zur Situation im Mundraum des Patienten auszuformen.

Seit einiger Zeit wird statt diesem Verfahren auch ein anderes Verfahren eingesetzt, um ein Modell für den Kiefer und die Einbausituation bereitzustellen. Mit dem Verfahren der Stereolithographie wird die dreidimensionale Situation im Mundraum des Patienten zunächst aufgenommen und in einem elektronischen Speicher eines Rechners digital hinterlegt. Dazu werden meist CAD-Programme verwendet. Anschließend wird ein Modell des Kiefers mit einem lichthärtenden Kunststoff in bekannter Weise mit Hilfe eines Lasers erzeugt. In diese Kunststoffmodelle des Kiefers werden dann die Laborimplantate eingesetzt, die auch für die Gipsmodelle verwendet werden. Das Kunststoffmodell des Kiefers kann dann wieder zum Aufbau des Zahnersatzes durch den Zahntechniker verwendet werden.

In beiden Fällen wird in das Modell des Kiefers also ein Laborimplantat eingesetzt, dass einen zylindrischen Bereich mit kreisrundem Querschnitt umfasst. In dem zylindrischen Bereich können Unterschnitte vorgesehen sein. Auf der Oberseite des Laborimplantats wird der Zahnersatz geformt. Der zylindrische Bereich steckt in einer entsprechenden Ausnehmung in dem Modell des Kiefers. Das Kopfteil des Laborimplantats ist so ausgeformt, wie der Kopf des Aufbauteils des Dentalimplantats, das dann direkt beim Patienten eingesetzt wird. So ist sichergestellt, dass der Zahnersatz genau auf den Kopf des Dentalimplantats passt.

Nachteilig ist hieran, dass die Einbausituation am Modell des Kiefers für den Zahntechniker nicht exakt definiert ist. So kann es beim Einsetzen des Zahnersatzes durch den Zahnarzt dazu kommen, dass der Zahnersatz nicht genau zu der Situation im Mundraum des Patienten passt. Dann muss der Zahnarzt den Zahnersatz noch vor Ort anpassen oder schlimmstenfalls muss der Zahnersatz komplett neu gefertigt werden. Das Laborimplantat lässt sich zudem nicht ohne weiteres in der richtigen Position fixieren, so dass es auch während der Herstellung des Zahnersatzes zu Positionsveränderungen und damit zu einem fehlerhaften Zahnersatz kommen kann. Das Laborimplantat ist nämlich drehbar und in seiner Längsrichtung verschiebbar in dem Modell angeordnet. Bekannt aus WO 2012/095851 A1 ist ein Verfahren zur Herstellung eines dreidimensionalen Modells, in welchem das Laborimplantat passgenau in die Ausnehmung eingeschraubt wird. Ebenfalls bekannt aus diesem Dokument ist ein entsprechendes Kiefermodell und ein entsprechendes Laborimplantat.

Die Aufgabe der Erfindung besteht also darin, die Nachteile des Stands der Technik zu überwinden. Insbesondere soll eine Möglichkeit gefunden werden, einen Zahnersatz mit Hilfe eines Modells des Kiefers, insbesondere eines mit Stereolithographie gefertigten Kunststoffmodells des Kiefers möglichst exakt herzustellen, so dass ein geringerer Ausschuss bei der Herstellung eines Zahnersatzes entsteht und dass der Zahnarzt weniger, beziehungsweise keine zusätzlichen Anpassungen mehr beim Einsetzen des Zahnersatzes vornehmen muss. Zudem sollen die modernen Verfahren bei der Herstellung des Modells des Kiefers anwendbar sein. Gleichzeitig sollen aber auch möglichst viele der Dentalimplantate einsetzbar sein, die sich derzeit auf dem Markt befinden. Es sollen ein Verfahren und zur Umsetzung des Verfahrens geeignete Vorrichtungen bereitgestellt werden.

Die Aufgabe der Erfindung wird durch ein Verfahren mit den Merkmalen des Anspruchs 1 gelöst. Eine zylindrische oder konische Ausnehmung wird in dem Modell erzeugt, in die das Laborimplantat eingesetzt wird, um das Laborimplantat an dem Modell zu positionieren, wobei die Ausnehmung mit einer einzähligen oder mehrzähligen Drehsymmetrie um die Längsachse der Ausnehmung erzeugt wird und ein Laborimplantat verwendet wird, das zumindest bereichsweise die gleiche Drehsymmetrie hat und so in die Ausnehmung passt, oder in der Ausnehmung ein Gegengewinde erzeugt wird, das zu einem Gewinde am Laborimplantat passt.

Unter einer zylindrischen Geometrie ist vorliegend ein zylindrischer Körper mit einer beliebigen Grundfläche zu verstehen. Eine konische Symmetrie ist vorliegend nicht nur als Kegel zu verstehen, sondern alle Geometrien, bei denen sich der Bereich vom Kopfteil zum Aufbau des Zahnersatzes weg verjüngt. Eine stetige Verjüngung ist nicht Voraussetzung für eine konische Symmetrie im Sinne der vorliegenden Erfindung, ist jedoch erfindungsgemäß bevorzugt, da sich solche Laborimplantate besser in die Ausnehmungen der Modelle der Kiefer einbringen lassen. Zudem können an der Oberfläche der Bereiche des Laborimplantats auch zusätzliche Strukturen, wie beispielsweise Rastungen, Unterschnitte und/oder Aufrauhungen vorgesehen sein, die die zylindrische oder konische Symmetrie streng genommen brechen. Die Ausnehmungen mit Gegengewinde sind vorzugsweise zylindrisch mit kreisrunder Grundfläche und zur Verwendung von Laborimplantaten mit einem kreisrunden zylindrischen Bereich und mit passendem Gewinde vorgesehen.

Die Längsachse der Ausnehmung ist die Achse entlang der das Laborimplantat in die Ausnehmung ein- und ausgeführt werden kann. Sie liegt meist auf der Mittelachse der Ausnehmung. Auch die zylindrische oder konische Grundform kann die Symmetrieachse vorgeben. Im Falle von einzähligen Drehsymmetrieachsen ist diejenige einzählige Drehsymmetrieachse gemeint, die mit der Drehsymmetrieachse der konischen oder zylindrischen Grundform zusammenfällt. Meist wird als Grundform des Bereichs des Implantats und damit der Ausnehmung nämlich eine zylindrische oder konische Form gewählt, bei der dann durch Rillen, Vertiefungen oder Überstände die Symmetrie dieser Grundform gebrochen wird. Als Symmetrieachse sei dann also die Drehsymmetrieachse der Grundform zu verstehen.

Es kann auch vorgesehen sein, dass als letzter Schritt des Verfahrens der Zahnersatz auf einem Kopfteil des Laborimplantats oder auf einem Abutment auf dem Laborimplantat aufgebaut wird.

Bei erfindungsgemäßen Verfahren kann bevorzugt auch vorgesehen sein, dass zur Herstellung der Ausnehmung, vorzugsweise des Modells mit der Ausnehmung ein Computer verwendet wird, insbesondere eine Digitalisierung des Mundraums des Patienten, wobei die Form der Ausnehmung aus einer Speichereinheit des Computers ausgelesen wird und wobei insbesondere verschiedenen Laborimplantaten verschiedene passende Formen der Ausnehmungen zugeordnet werden, die vorzugsweise durch den Computer und/oder einen Anwender unter Verwendung einer Eingabeeinheit ausgewählt werden.

Bei der Verwendung eines Computers zur Herstellung des Modells ergibt sich der besondere kombinatorische Effekt, dass in dem Computer schon alle Daten zur Herstellung des Modells gespeichert sind und daher die Oberfläche des Modells gespeichert ist. Hier muss die Oberfläche des Modells des Kiefers nur noch derart modifiziert werden, dass dort nun an der richtigen Stelle die Ausnehmung angeordnet wird, die zu dem Laborimplantat passt, welches am besten geeignet ist oder das vom Computer zuvor ausgewählt wurde oder das als einziges gespeichert ist. Das Modell kann dann gleich mit der modifizierten Oberfläche erzeugt werden, das heißt zusammen mit der passenden Ausnehmung erzeugt werden.

Es kann auch vorgesehen sein, dass ein Laborimplantat verwendet wird, das zumindest bereichsweise entlang seiner Längsachse die gleiche Drehsymmetrie wie die Ausnehmung aufweist und das vorzugsweise passgenau in die Ausnehmung passt oder dessen Gewinde genau zum Gegengewinde der Ausnehmung passt.

Bei der Verwendung eines solchen Laborimplantats ist sichergestellt, dass die Ausnehmung zu dem Bereich des Laborimplantats passt und daher das Laborimplantat gut in dem Modell befestigt ist.

Ferner kann vorgesehen sein, dass zur Herstellung des Modells ein CAD-gestütztes stereolithographisches Verfahren verwendet wird und das Modell vorzugsweise aus Kunststoff, besonders bevorzugt aus einem lichthärtenden Kunststoff gefertigt wird.

Solche Verfahren sind besonders gut zur Realisierung erfindungsgemäßer Verfahren geeignet, da sich die Ausnehmungen so sehr einfach und ohne großen Rechenaufwand auch mit einfachen, leicht erhältlichen Computern in die Modelle des Kiefers integrieren lassen.

Dabei kann vorgesehen sein, dass bei der Herstellung des Modells eine fest vorgegebene Form für die Ausnehmung vorgegeben wird, die zu einem bestimmten Laborimplantat passt, und/oder eine Form für die Ausnehmung aus einer Mehrzahl verschiedener Formen ausgewählt wird, die zu einer Mehrzahl verschiedener Laborimplantate passen, wobei vorzugsweise vor der Herstellung des Modells die Form in ein gemessenes virtuelles digitalisiertes Modell des Kiefers als Ausnehmung integriert wird.

Hierdurch lässt sich ein besonders anwenderfreundliches und/oder variables Verfahren gestalten. Die Stärke der Erfindung lässt sich gerade mit solchen Möglichkeiten nutzbringend umsetzen.

Erfindungsgemäß ist vorsehen, dass das Laborimplantat passgenau in die Ausnehmung eingeschraubt wird und mit einer Rastung in eine Gegenrastung in der Ausnehmung einrastet.

Hierdurch wird ganz besonders eine rotationsfeste und/oder positionsfeste Anordnung des Laborimplantats im Modell erreicht und dadurch die Möglichkeit zum Aufbau eines qualitativ hochwertigen Zahnersatzes gegeben.

Auch kann vorgesehen sein, dass die Herstellung des Modells und der Ausnehmung in einem Schritt erfolgt, wobei vorzugsweise die äußere Form des Modells aufgrund einer Messung am Patienten erfolgt und die Form der Ausnehmung fest vorgegeben wird.

Das Einsparen eines zusätzlichen Arbeitsschritts erspart Kosten und Zeit bei der Herstellung.

Ferner ist erfindungsgemäß vorgesehen, dass in der Ausnehmung ein Gegengewinde mit Anschlag, das zu einem Gewinde am Laborimplantat passt, in dem Modell erzeugt wird, wobei auch das Gewinde des Laborimplantats einen Anschlag umfasst.

Auch diese Maßnahme dient der besseren Positionierung und Fixierung des Laborimplantats.

Die Aufgabe der Erfindung wird auch gelöst durch ein Modell eines Kiefers zur Herstellung eines Zahnersatzes nach Anspruch 8, insbesondere zum Durchführen eines solchen Verfahrens umfassend eine Ausnehmung zum Einsetzen eines Laborimplantats, bei dem die Ausnehmung zylindrisch oder konisch geformt ist und eine einzählige oder mehrzählige Drehsymmetrieachse entlang der Längsachse hat, so dass ein Laborimplantat mit zumindest bereichsweise gleicher Drehsymmetrie rotationsfest in der Ausnehmung anzuordnen ist, oder in der Ausnehmung ein Gegengewinde, vorzugsweise mit einem Anschlag, vorgesehen ist, das zu einem Gewinde, vorzugsweise mit Anschlag, eines Laborimplantats passt, so dass das Laborimplantat positionsfest in dem Modell anzuordnen ist.

Ein solches Modell liefert eine Basis für die Verwendung eines rotationsfesten oder sogar positionsfesten Aufbaus zur Herstellung eines qualitativ hochwertigen Zahnersatzes.

Dabei ist erfindungsgemäß vorgesehen, dass in der Ausnehmung eine Gegenrastung vorgesehen ist, die in eine Rastung an dem Laborimplantat greift und das Laborimplantat positionsfest hält.

Dies dient wieder dazu, ein Laborimplantat, das in die Ausnehmung des Modells eingesetzt wird, besser und genauer zu fixieren.

Die Aufgabe der Erfindung wird ferner gelöst durch ein Laborimplantat zur Herstellung eines Zahnersatzes nach Anspruch 9, insbesondere zum Durchführen eines der beschriebenen Verfahren, umfassend einen Bereich zum Einstecken in eine Ausnehmung eines Modells eines Kiefers und einen Kopfteil zum Aufbau des Zahnersatzes, wobei der Bereich zylindrisch oder konisch geformt ist und eine einzählige oder mehrzählige Drehsymmetrieachse um die Längsachse des Bereichs aufweist, so dass das Laborimplantat rotationsfest in einer Ausnehmung mit passender Form anzuordnen ist oder dass der Bereich ein Außengewinde umfasst, vorzugsweise ein Außengewinde mit Anschlag, so dass das Laborimplantat in einer Ausnehmung mit passendem Innengewinde positionsfest anzuordnen ist.

Ein solches Laborimplantat führt bei seiner Verwendung, insbesondere zusammen mit einem erfindungsgemäßen Modell, zu einer Umsetzung der Erfindung. Durch die Abweichung von der Rotationssymmetrie um die Längsachse des Bereichs des Laborimplantats, der eingesteckt wird, wird sichergestellt, dass der Zahnersatz, der auf dem Kopfteil / der Oberseite des Laborimplantats aufgebaut wird, stets in der richtigen Position zum Modell des Kiefers aufgebaut wird und auch später im richtigen Winkel bezüglich der Längsachse des Implantats im Mundraum des Patienten eingesetzt wird. Die gleiche Argumentation kann auch bezüglich der Vorteile der nicht rotationssymmetrischen Ausnehmung im Modell des Kiefers verwendet werden.

Dabei ist erfindungsgemäß vorgesehen, dass das Laborimplantat eine Rastung zum Einrasten in eine Gegenrastung in der Ausnehmung umfasst, die das Laborimplantat positionsfest in der Ausnehmung hält.

Dies dient wieder der Verbesserung der Fixierung des Laborimplantats im Modell des Kiefers und damit der Verbesserung der Qualität des Zahnersatzes.

Ferner kann vorgesehen sein, dass das Laborimplantat in dem Bereich eine Vertiefung in Längsrichtung aufweist.

Eine solche Vertiefung ist zur Realisierung eines besonders einfach aufgebauten Implantats mit einzähliger oder auch mehrzähliger Drehsymmetrie um die Längsachse geeignet, der sich leicht einsetzen aber auch wieder aus dem Model entfernen lässt.

Gemäß einer weiteren Ausgestaltung der Erfindung kann vorgesehen sein, dass das Laborimplantat ein Befestigungsmittel zum Befestigen eines Aufbaus oder Abutments umfasst.

Der Zahnersatz kann dann auf dem, in dem Laborimplantat befestigten Aufbau oder Abutment aufgebaut werden. Dadurch wird die Aufbausituation stabilisiert.

Ferner kann vorgesehen sein, dass das Laborimplantat in dem Bereich Unterschnitte zum Aufnehmen eines Zements umfasst, so dass das Laborimplantat bifunktional auch mit Zementier-Technik einsetzbar ist.

Ein solches Laborimplantat ist dann nicht nur mit einem erfindungsgemäßen Verfahren einsetzbar, sondern kann auch für die herkömmlichen Verfahren zur Herstellung eines Zahnersatzes verwendet werden, bei denen das Laborimplantat in dem Modell einzementiert wird und die Unterschnitte dazu dienen, dass der Zement eine feste Verbindung mit dem Laborimplantat bildet. Solche Laborimplantate sind dann universell einsetzbar, so dass eine einzige Produktlinie ausreicht, um den Markt zu versorgen.

Eine mögliche Anwendung verschiedener Laborimplantate und die Auswahl eines besonders geeigneten Laborimplantats eröffnet die Möglichkeit zu einer weiteren Verbesserung des erzeugten Endergebnisses.

Der Erfindung liegt die überraschende Erkenntnis zugrunde, dass bei der Anwendung moderner Verfahren, wie "Rapid-Prototyping" und/oder stereolithographischen Verfahren, zur Herstellung von Modellen von Kiefern eine Verbesserung der Qualität des Zahnersatzes möglich ist, wenn Laborimplantate mit Bereichen zum Einbringen in die Modelle mit nicht kreisrundem Querschnitt verwendet werden und diese Symmetrie auch durch die Ausnehmung im Modell realisiert wird, in die das Laborimplantat eingesetzt wird. Durch verschiedene erfindungsgemäße Maßnahmen kann es dabei gelingen, das Laborimplantat besonders ortsgenau, das heißt, rotationsfest oder sogar positionsfest im Modell anzuordnen.

Zudem kann die passende Ausnehmung gleich beim Erzeugen des Modells in dem Modell des Kiefers erzeugt werden, wenn die für die Ausnehmung notwendige Oberfläche gleich in ein virtuelles Modell integriert wird und beim Herstellen das Modell gleich entsprechend erzeugt wird. Wenn beides *in einem* Arbeitsschritt erfolgt muss nicht erst das Modell erzeugt werden, um dann anschließend wieder Material für die Herstellung der Ausnehmung abzutragen. Außerdem kann auch gleich eine besonders gut passende Ausnehmung mit einem dazu passenden Laborimplantat ausgewählt werden, wenn mehrere verschiedene Laborimplantate und die dazu passenden Ausnehmungen gespeichert sind und so zur Auswahl stehen.

Im Folgenden werden Ausführungsbeispiele der Erfindung anhand von vier schematisch dargestellten Figuren erläutert, ohne jedoch dabei die Erfindung zu beschränken. Zusätzlich wird eine Figur zu einem Laborimplantat nach dem Stand der Technik gezeigt. Dabei zeigt:
- Figur 1:: eine schematische Querschnittansicht eines Laborimplantats nach dem Stand der Technik;
- Figur 2:: eine schematische, perspektivische Ansicht eines Laborimplantats;
- Figur 3:: eine schematische, perspektivische Ansicht eines zweiten Laborimplantats;
- Figur 4:: einen Ausschnitt aus einer schematischen Querschnittansicht eines erfindungsgemäßen Modells eines Kiefers mit eingesetztem Laborimplantat; und
- Figur 5:: schematische Querschnitte durch vier verschiedene Laborimplantate senkrecht zu deren Längsachse.

Figur 1 zeigt eine schematische Querschnittansicht eines Laborimplantats 1 nach dem Stand der Technik, wobei sich dessen Längsachse waagerecht in der Mitte des Querschnitts befindet. Das Laborimplantat 1 umfasst einen Kopfteil 2 und einen hinteren Bereich 3, der in das Modell eines Kiefers eingesetzt werden kann. Das Kopfteil 2 dient dazu, den eigentlichen Zahnersatz (nicht gezeigt) auf diesem Kopfteil 2 aufzubauen. Dazu wird das Laborimplantat 1 in ein Modell eines Kiefers eines Patienten eingebettet, so dass der aufzubauende Zahnersatz zur Situation im Mundraum des Patienten passt. Dazu wird der das Laborimplantat 1 in das Modell eingesetzt und dort in das Gips-Modell integriert. Damit der Gips das Laborimplantat 1 gut befestigt, sind im hinteren Bereich 3 Unterschnitte 4 vorgesehen, die sich mit dem Gips füllen und so für eine stabile Verbindung des Laborimplantats 1 mit dem Modell sorgen. Das Laborimplantat 1 hat zylindrische Geometrie mit kreisrunder Grundfläche, das heißt, dass alle Querschnitte senkrecht zur Längsachse des Laborimplantats 1 (also senkrecht in der Bildebene nach Figur 1) kreisrund sind.

Solche Laborimplantate 1, wie in Figur 1 gezeigt, werden auch dazu verwendet, Zahnersatz bei der Verwendung von Kunststoffmodellen aufzubauen. Solche Kunststoffmodelle werden beispielsweise durch Scannen des Mundraums, beziehungsweise des betroffenen Teils des Mundraums des Patienten und anschließendem automatisiertem Aufbau des Kunststoffmodells erzeugt. Beispielsweise kann Stereolithographie eingesetzt werden, um solche Modelle zu formen.

Bei einem so aufgebauten Zahnersatz kann sich jedoch das Problem ergeben, dass diese nicht genau zu der Einbausituation im Mundraum des Patienten passen. Dazu wurde im Rahmen der vorliegenden Erfindung festgestellt, dass die Laborimplantate 1 aufgrund ihrer Rotationssymmetrie um Ihre Längsachse (in Figur 1 als eine waagerechte Linie in der Mitte des Schnitts des Laborimplantats 1 zu denken) in den Ausnehmungen des Modells drehbar sind, wodurch es zu solchen Abweichungen beim Aufbau des Zahnersatzes kommen kann.

Figur 2 zeigt eine schematische perspektivische Ansicht eines Laborimplantats 11, das dieses Problem löst. Das Laborimplantat 11 umfasst ein Kopfteil 12 und einen zylindrischen Bereich 13, der in das Kunststoffmodell eingesteckt werden kann. Der zylindrische Bereich 13 hat eine zylindrische Geometrie mit nicht kreisrunder Grundfläche. Der Zylindermantel weist eine Längsrille 14 auf, die die Runde Symmetrie des zylindrischen Bereichs 13 bricht. Der zylindrische Bereich 13 des Laborimplantats 11 hat dadurch eine einzählige Drehsymmetrie um die Zylinderachse, das heißt, dass der zylindrische Bereich 13 nur bei einer Drehung um 360° um diese Zylinderachse in seinen Ausgangszustand überführt werden kann. Dies führt dazu, dass das Laborimplantat 11 nur in einer Position eine Ausnehmung eines Modells eines Kiefers (nicht gezeigt) eingesteckt werden kann. Dadurch wird die Position des Laborimplantats 11 rotatorisch fixiert.

Ein Zahnersatz (nicht gezeigt), der auf einem dermaßen fixierten Laborimplantat 11 aufgebaut wird, wird dann auch im Mundraum beim Patienten genau in dem gleichen Winkel aufgesetzt werden, da das Kopfteil 12 des Laborimplantats 11 genau die gleiche Ausformung hat, wie das Kopfteil des tatsächlichen Dentalimplantats. Bei der Herstellung des Zahnersatzes wird der Zahnersatz stets in der gleichen Position im Modell gehalten und kann sich nicht während der Herstellung verdrehen. Auch der Kopfteil 12 eines erfindungsgemäßen Laborimplantats 11 kann vorteilhaft von einer Drehsymmetrie abweichen. Es können jedoch auch alle anderen Kopfteile 12, die derzeit bei den auf dem Markt befindlichen Dentalimplantaten vorkommen realisiert werden. Im Kopfteil kann eine Bohrung mit einem Innengewinde oder ein anderes Befestigungsmittel (nicht gezeigt) vorgesehen sein, auf dem ein Abutment befestigt werden kann.

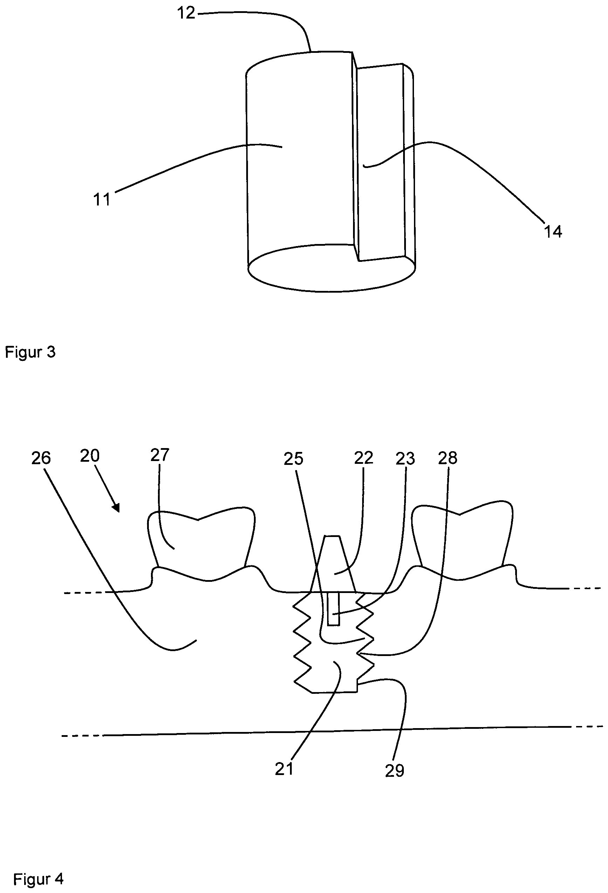

Figur 3 zeigt eine schematische perspektivische Ansicht eines zweiten Laborimplantats 11, wobei das Laborimplantat 11 hier durchgehend zylindrische Symmetrie mit einer Ausnehmung 14 hat. Die Oberseite 12 ist hier der Kopfteil 12 des Laborimplantats 11 und der Bereich, der in ein Modell eines Kiefers eingebracht werden kann, kann hier das Laborimplantat 11 auf seiner ganzen Länge sein. Das Laborimplantat 11 kann in eine Ausnehmung eines Modells eines Kiefers mit einer entsprechend geformten Ausnehmung in genau einer Position eingesetzt werden. Die Ausnehmung bildet das Negativ des Laborimplantats 11, muss aber nicht die gleiche Höhe haben. Statt eines zylindrischen Laborimplantats 11 kann auch ein konisches Laborimplantat verwendet werden, dass sich vom Kopfteil 12 weg verjüngt.

Figur 4 zeigt eine schematische Querschnittansicht eines Ausschnitts eines erfindungsgemäßen Modells 20 eines Kiefers mit einem eingesetzten erfindungsgemäßen Laborimplantat 21. Auf der Oberseite des Laborimplantats 21 ist ein Aufbau 22 angeordnet, das mit einem Befestigungsmittel 23 mit dem Laborimplantat 21 verbunden ist. Auf dem Aufbau 22 kann der Zahnersatz aufgebaut werden. Das Laborimplantat 21 wird in eine Ausnehmung des Modells 20 entlang deren Längsachsen eingebracht. Das Laborimplantat 21 ist zylindrisch geformt und umfasst ein Außengewinde 25.

Das Modell 20 umfasst einen Kiefersattel 26 und Zähne 27, beziehungsweise genaugenommen Modelle davon. Das Modell 20 ist aus Kunststoff gefertigt und wurde mit einem stereolithographischen Verfahren erzeugt. Der gegenüberliegende Kieferteil (nicht gezeigt) ist ebenfalls Teil des Modells 20, um die Situation beim Kauen, beziehungsweise bei geschlossenem Mund darstellen zu können. Das Modell 20 des Kiefers setzt sich also nicht nur nach rechts und links fort, wie durch die gestrichelten Linien angedeutet.

Das Modell 20 umfasst an einer Stelle zwischen den Zähnen 27 eine Ausnehmung in Form eines zylindrischen Lochs. An den Innenwänden der Ausnehmung ist ein Innengewinde 28 vorgesehen, das zu dem Außengewinde 25 des Laborimplantats 21 passt. Die Gewinde 25, 27 oder zumindest eines der Gewinde 25, 27 schließt mit einem Anschlag 29 ab. Der Anschlag 29 dient dazu, dass die Position des Laborimplantats 21 im Modell 20 definiert ist. Dadurch ist die genaue Position des Laborimplantats 21 im Modell 20 fixiert, das heißt, sowohl rotationsfest, als auch positionsfest.

Für ein Laborimplantat 11, wie es in Figur 2 oder 3 gezeigt ist, wäre die Ausnehmung in einem passenden Modell als zylindrisches Loch mit einem Vorsprung aufgebaut, der in die Längsrille 14 greift und damit das Laborimplantat 11 rotationsfest hält. Der Vorzug dieses Aufbaus ist darin zu sehen, dass das Laborimplantat 11 schnell herausnehmbar ist, während das Laborimplantat 21 nach Figur 4 besser und genauer fixierbar ist.

Im Folgenden wird ein Ausführungsbeispiel eines erfindungsgemäßen Verfahrens erläutert. Der Mundraum eines Patienten wird vermessen, um einen passenden Zahnersatz zu erzeugen. Dazu wird ein dreidimensionales Bild des Mundraums, beziehungsweise insbesondere des betroffenen Teils des Kiefers aufgenommen. Diese Daten werden in dem Speicher eines Computers gespeichert und verarbeitet.

Aus den Daten wird ein virtuelles dreidimensionales CAD-Modell des Kiefers oder des Teils des Kiefers in einem Rechner des Computers berechnet. In dem Speicher des Computers sind verschiedene Ausnehmungen gespeichert, die verschiedenen Laborimplantaten 11, 21 zugeordnet sind. Der Computer berechnet, wie die verschiedenen Ausnehmungen in dem Modell 20 angeordnet werden könnten. Die stabilste oder sinnvollste Variante wird ausgewählt oder die stabilsten oder sinnvollsten Varianten werden über eine Anzeigeeinrichtung vorgeschlagen. Als Kriterium kann beispielsweise verwendet werden, wie dick die Wandstärke des Modells 20 im Bereich der Ausnehmung in dem Kiefersattel 26 für die verschiedenen Ausnehmungen sind oder welches für die Position im Kiefer das geeignetste Laborimplantat 11, 21 ist.

Der Anwender kann auch alternativ aus allen vorhandenen gespeicherten Ausnehmungen diejenige auswählen, die er für an geeignetsten hält. Wobei auch vorgesehen sein kann, dass der Computer durch Berechnung darauf hinweist wenn eine ausgewählte Ausnehmung nicht möglich oder nicht sinnvoll, also zum Beispiel nicht stabil genug erscheint. Ebenso kann auch nur eine mögliche Form einer Ausnehmung für eine bestimmte Sorte von Laborimplantaten 11, 21 gespeichert sein.

Anschließend wird die über eine Eingabeeinrichtung die ausgewählte Ausnehmung oder die einzige vorgegebene Ausnehmung mit dem virtuellen Modell des Kiefers kombiniert. Dazu wird die Ausnehmung als Oberfläche des virtuellen Modells an der Stelle in das virtuelle Modell des Kiefers gerechnet, an dem das Dentalimplantat im Kiefer des Patienten angeordnet ist. Aus dem neuen virtuellen Modell wird dann ein tatsächliches Modell 20 mit bekannten Verfahren, wie zum Beispiel "Rapid-Prototyping"-Verfahren erzeugt. Hierzu eignen sich insbesondere stereolithographische Verfahren. Dazu ist der Computer an eine Herstellungseinrichtung angeschlossen, mit der solche Verfahren umsetzbar sind.

In die Ausnehmung des hergestellten Modells 20 kann das dieser Ausnehmung zugeordnete Laborimplantat 11, 21 eingesetzt werden. Bezüglich Figur 4 kann das Laborimplantat 21 in die Ausnehmung des Modells 20 eingeschraubt werden. Auf dem Aufbau 22 beziehungsweise dem Abutment 22 des Laborimplantats 11, 21 kann dann der eigentliche Zahnersatz in einer realistischen Umgebung erzeugt werden. Immer wenn das Laborimplantat 11, 21 aus dem Modell 20 entfernt und wieder eingesetzt wird, wird es sich an der gleichen Position im Modell 20 des Kiefers befinden. Damit kann sichergestellt werden, dass der Zahnersatz genau die richtige Form hat. Zudem wird der Zahnersatz auch in der gleichen Position im Mundraum des Patienten auf dem eigentlichen Dentalimplantat angeordnet sein, so dass eine besonders gute Passform sichergestellt ist.

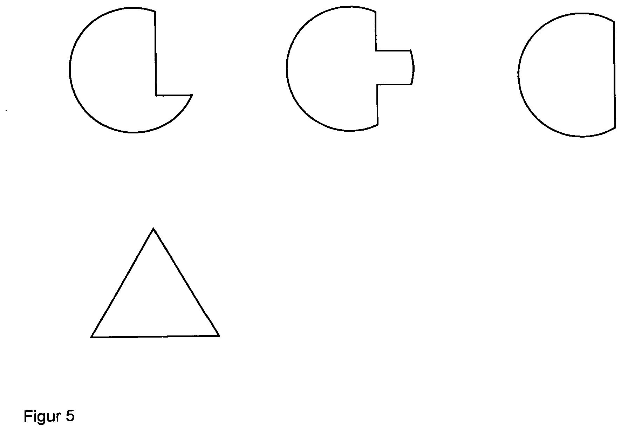

In Figur 5 werden als schematische Querschnittansichten die Querschnitte von vier verschiedenen Bereichen von Laborimplantaten gezeigt, die zum Einbringen in Modelle von Kiefern vorgesehen sind. Die Bereiche sind entlang der Längsachse geschnitten sind. Die ersten drei Schnitte zeigen eine kreisrunde Basis, mit einer oder zwei Ausnehmungen, so dass die Drehsymmetrie um die Längsachse gebrochen wird. Alle gezeigten Bereiche der Laborimplantate haben so eine einzählige Drehsymmetrie um die Längsachse. Die Bereiche können entweder zylindrisch oder auch konisch sein.

Wenn stattdessen ein Laborimplantat mit einem Bereich mit einem sechseckigen Querschnitt verwendet wird, hat der Bereich und/oder die Ausnehmungen hierzu eine sechszählige Drehsymmetrie, das heißt, dass der Bereich und die Oberfläche der Ausnehmung durch sechs verschiedene Drehungen um die Längsachse von kleiner gleich 360° geometrisch in sich selbst überführt werden kann. Ein rechteckiger oder elliptischer Querschnitt würde zu einer zweizähligen Symmetrie führen.

Die vierte schematische Querschnittansicht nach Figur 5 zeigt ein Laborimplantat mit einem Bereich mit dreieckigem Querschnitt, also mit dreizähliger Drehsymmetrie. Das Laborimplantat kann in eine Ausnehmung mit gleicher dreizähliger Drehsymmetrie gesteckt werden.

### Bezugszeichenliste

- 1, 11, 21: Laborimplantat
- 2, 12: Kopfteil
- 3: Hinterer Bereich
- 4: Unterschnitt
- 13: Zylindrischer Bereich
- 14: Längsrille
- 20: Modell
- 22: Aufbau / Abutment
- 23: Befestigungsmittel
- 25: Außengewinde
- 26: Kiefersattel
- 27: Zahn
- 28: Innengewinde
- 29: Anschlag

## Patentansprüche

1. Verfahren zur Herstellung eines dreidimensionalen Modells (20) zumindest eines Teilbereichs eines Kiefers zum Herstellen eines Zahnersatzes, bei dem ein Laborimplantat (11, 21) in einer Ausnehmung des Modells (20) angeordnet wird, wobei eine zylindrische oder konische Ausnehmung in dem Modell (20) erzeugt wird, in die das Laborimplantat (11, 21) eingesetzt wird, um das Laborimplantat (11, 21) an dem Modell (20) zu positionieren, wobei in der Ausnehmung ein Gegengewinde (28) mit Anschlag (29) erzeugt wird, das zu einem Gewinde (25) am Laborimplantat (11, 21) passt, wobei auch das Gewinde (25) des Laborimplantats (11, 21) einen Anschlag (29) umfasst, und wobei das Laborimplantat (11, 21) passgenau in die Ausnehmung eingeschraubt wird, **dadurch gekennzeichnet, dass** das Laborimplantat mit einer Rastung in eine Gegenrastung in der Ausnehmung einrastet.

2. Verfahren nach Anspruch 1, **dadurch gekennzeichnet, dass**
zur Herstellung der Ausnehmung des Modells (20) mit der Ausnehmung, ein Computer verwendet wird, insbesondere eine Digitalisierung des Mundraums des Patienten, wobei die Form der Ausnehmung aus einer Speichereinheit des Computers ausgelesen wird und wobei verschiedenen Laborimplantaten (11, 21) verschiedene passende Formen der Ausnehmungen zugeordnet werden, die durch den Computer und/oder einen Anwender unter Verwendung einer Eingabeeinheit ausgewählt werden.

3. Verfahren nach einem der Ansprüche 1 oder 2, **dadurch gekennzeichnet, dass**
ein Laborimplantat (11, 21) verwendet wird, das zumindest bereichsweise entlang seiner Längsachse die gleiche Drehsymmetrie wie die Ausnehmung aufweist und das passgenau in die Ausnehmung passt oder dessen Gewinde (25) genau zum Gegengewinde (28) der Ausnehmung passt.

4. Verfahren nach einem der vorangehenden Ansprüche, **dadurch gekennzeichnet, dass** zur Herstellung des Modells (20) ein CAD-gestütztes stereolithographisches Verfahren verwendet wird und das Modell (20) aus einem lichthärtenden Kunststoff gefertigt wird.

5. Verfahren nach einem der vorangehenden Ansprüche, **dadurch gekennzeichnet, dass** bei der Herstellung des Modells (20) eine fest vorgegebene Form für die Ausnehmung vorgegeben wird, die zu einem bestimmten Laborimplantat (11, 21) passt, und/oder eine Form für die Ausnehmung aus einer Mehrzahl verschiedener Formen ausgewählt wird, die zu einer Mehrzahl verschiedener Laborimplantate (11, 21) passen.

6. Verfahren nach Anspruch 5, **dadurch gekennzeichnet, dass** vor der Herstellung des Modells (20) die Form in ein gemessenes virtuelles digitalisiertes Modell des Kiefers als Ausnehmung integriert wird.

7. Verfahren nach einem der vorangehenden Ansprüche, **dadurch gekennzeichnet, dass** die Herstellung des Modells (20) und der Ausnehmung in einem Schritt erfolgt, wobei die äußere Form des Modells (20) aufgrund einer Messung am Patienten erfolgt und die Form der Ausnehmung fest vorgegeben wird.

8. Modell eines Kiefers zur Herstellung eines Zahnersatzes zum Durchführen eines Verfahrens nach einem der vorangehenden Ansprüche, umfassend eine Ausnehmung zum Einsetzen eines Laborimplantats (11, 21), wobei
die Ausnehmung zylindrisch oder konisch geformt ist und in der Ausnehmung ein Gegengewinde (28) mit einem Anschlag (29) vorgesehen ist, das zu einem Gewinde (25) mit Anschlag (29) eines Laborimplantats (11, 21) passt, so dass das Laborimplantat (11, 21) positionsfest in dem Modell (20) anzuordnen ist, **dadurch gekennzeichnet, dass** in der Ausnehmung eine Gegenrastung vorgesehen ist, die in eine Rastung an dem Laborimplantat (11, 21) greift und das Laborimplantat (11, 21) positionsfest hält.

9. Laborimplantat zur Herstellung eines Zahnersatzes zum Durchführen eines Verfahrens nach einem der Ansprüche 1 bis 7, umfassend einen Bereich (13) zum Einstecken in eine Ausnehmung eines Modells (20) eines Kiefers und einen Kopfteil (12) zum Aufbau des Zahnersatzes, wobei
der Bereich (13) zylindrisch oder konisch geformt ist und dass der Bereich ein Außengewinde (25) mit Anschlag (29) umfasst, so dass das Laborimplantat (11, 21) in einer Ausnehmung mit passendem Innengewinde (28) positionsfest anzuordnen ist, **dadurch gekennzeichnet, dass** das Laborimplantat (11, 21) eine Rastung zum Einrasten in eine Gegenrastung in der Ausnehmung umfasst, die das Laborimplantat (11, 21) positionsfest in der Ausnehmung hält.

10. Laborimplantat nach Anspruch 9, **dadurch gekennzeichnet, dass** das Laborimplantat (11, 21) in dem Bereich (13) eine Vertiefung (14) in Längsrichtung aufweist.

11. Laborimplantat nach einem der Ansprüche 9 oder 10, **dadurch gekennzeichnet, dass** das Laborimplantat (11, 21) ein Befestigungsmittel (23) zum Befestigen eines Aufbaus (22) oder Abutments (22) umfasst.

12. Laborimplantat nach einem der Ansprüche 9 bis 11, **dadurch gekennzeichnet, dass** das Laborimplantat (11, 21) in dem Bereich (13) Unterschnitte zum Aufnehmen eines Zements umfasst, so dass das Laborimplantat (11, 21) bifunktional auch mit Zementier-Technik einsetzbar ist.

## Claims

1. Method for the production of a three-dimensional model (20) of at least a partial region of a jaw for producing a dental prosthesis, in which a lab analog (11, 21) is arranged in a recess of the model (20),
a cylindrical or conical recess being created in the model (20) into which the lab analog (11, 21) is inserted to position the lab analog (11, 21) on the model (20), a mating thread (28) with a limit stop (29) being created in the recess fitting with a thread (25) on the lab analog (11, 21), the thread (25) of the lab analog (11, 21) also comprising a limit stop (29), and the lab analog (11, 21) being precisely screwed in into the recess, **characterised in that**
the lab analog engages with a lock-in position in a mating lock-in position in the recess.

2. Method according to claim 1, **characterised in that**
a computer is used for producing the recess of the model (20) having the recess, in particular a digitisation of the patient's oral cavity is used, the shape of the recess being read out of a memory unit of the computer, and various fitting shapes of the recesses being assigned to various lab analogs (11, 21), selected by the computer and/or by a user using an input unit.

3. Method according to any one of the claims 1 or 2, **characterised in that**
a lab analog (11, 21) is used having, at least in some regions, the same rotational symmetry along its longitudinal axis as the recess, and fitting precisely in the recess, or the thread (25) of which fitting precisely with the mating thread (28) of the recess.

4. Method according to any one of the preceding claims, **characterised in that**
a CAD-assisted stereolithographic method is used to produce the model (20), and the model (20) is made out of a light-curing plastic.

5. Method according to any one of the preceding claims, **characterised in that**
a predefined shape for the recess is predefined in the production of the model (20), fitting with a certain lab analog (11, 21), and/or a shape for the recess is selected from a plurality of different shapes, fitting with a plurality of different lab analogs (11, 21).

6. Method according to claim 5, **characterised in that** the shape is integrated as a recess into a measured virtual digitised model of the jaw before producing the model (20).

7. Method according to any one of the preceding claims, **characterised in that**
the production of the model (20) and the recess is carried out in one step, the external shape of the model (20) being based on a measurement on the patient, and the shape of the recess being predefined.

8. Model of a jaw for the production of a dental prosthesis for performing a method according to any one of the preceding claims, comprising a recess for inserting a lab analog (11, 21),
the recess having a cylindrical or conical shape, and a mating thread (28) with a limit stop (29) being provided in the recess fitting with a thread (25) with a limit stop (29) of a lab analog (11, 21), so that the lab analog (11, 21) is to be arranged in the model (20) in a positionally fixed manner, **characterised in that**
a mating lock-in position is provided in the recess, engaging in a lock-in position on the lab analog (11, 21) and holding the lab analog (11, 21) in a positionally fixed manner.

9. Lab analog for the production of a dental prosthesis for performing a method according to any one of claims 1 to 7, comprising a region (13) for inserting into a recess of a model (20) of a jaw and a head piece (12) for mounting the dental prosthesis,
the region (13) having a cylindrical or conical shape, and the region comprising an outside thread (25) with a limit stop (29), so that the lab analog (11, 21) is to be arranged in a recess having a fitting inside thread (28) in a positionally fixed manner, **characterised in that**
the lab analog (11, 21) comprises a lock-in position for engaging in a mating lock-in position in the recess holding the lab analog (11, 21) in the recess in a positionally fixed manner.

10. Lab analog according to claim 9, **characterised in that**
the lab analog (11, 21) has a recess (14) in the longitudinal direction in the region (13).

11. Lab analog according to any one of the claims 9 or 10, **characterised in that** the lab analog (11, 21) comprises a fastening means (23) for fastening a structure (22) or an abutment (22).

12. Lab analog according to any one of the claims 9 to 11, **characterised in that** the lab analog (11, 21) comprises undercuts for receiving a cement in the region (13), so that the lab analog (11, 21) is also bifunctionally insertable by means of a cementing technique.

## Revendications

1. Procédé pour la production d'un modèle tridimensionnel (20) d'au moins une région partielle d'une mâchoire pour produire une prothèse dentaire, dans lequel un analogue de laboratoire (11, 21) est disposé dans un évidement du modèle (20),
un évidment cylindrique ou conique étant créé dans le modèle (20), dans lequel l'analogue de laboratoire (11, 21) est inséré pour positionner l'analogue de laboratoire (11, 21) au modèle (20), un filetage d'accouplement (28) avec une butée (29) étant créé dans l'évidement concordant avec un filetage (25) à l'analogue de laboratoire (11, 21), le filetage (25) de l'analogue de laboratoire (11, 21) comprenant une butée (29) aussi, et l'analogue de laboratoire (11, 21) étant précisément vissé dans l'évidement, **caractérisé en ce que**
l'analogue de laboratoire s'enclenche avec une position de verrouillage dans une position de verrouillage d'accouplement dans l'évidement.

2. Procédé selon la revendication 1, **caractérisé en ce qu'**
un ordinateur est utilisé pour produire l'évidement du modèle (20) ayant l'évidement, en particulier une numérisation de la cavité buccale du patient est utilisée, la forme de l'évidement étant lue d'une unité de mémoire de l'ordinateur, et des formes concordantes différentes des évidements étant assignés à des analogues différents de laboratoire (11, 21), sélectionnées par l'ordinateur et/ou par un utilisateur utilisant une unité d'entrée.

3. Procédé selon l'une des revendications 1 ou 2, **caractérisé en ce qu'**
un analogue de laboratoire (11, 21) est utilisé ayant, au moins dans quelques régions, la même symétrie rotationnelle le long de l'axe longitudinale que l'évidement, et concordant précisément dans l'évidement, ou dont le filetage (25) concordant précisément avec le filetage d'accouplement (28) de l'évidement.

4. Procédé selon l'une des revendications précédentes, **caractérisé en ce qu'**
un procédé stéréolithographique assisté par CAO est utilisé pour produire le modèle (20), et le modèle est fabriqué en plastique photopolymérisable.

5. Procédé selon l'une des revendications précédentes, **caractérisé en ce qu'**
une forme prédéfinie pour l'évidement est prédéfinie lors de la production du modèle (20), concordant avec un analogue de laboratoire (11, 21) certain, et/ou une forme pour l'évidement est sélectionnée d'une pluralité de formes différentes, concordant avec une pluralité d' analogues de laboratoire (11, 21) différents.

6. Procédé selon la revendication 5, **caractérisé en ce que** la forme est intégrée comme un évidement dans un modèle numérisé virtuel mesuré de la mâchoire avant de produire le modèle (20).

7. Procédé selon l'une des revendications précédents, **caractérisé en ce que**
la production du modèle (20) et de l'évidement est effectuée en une étape, la forme externe du modèle (2) reposant sur une mesure au patient, et la forme de l'évidement étant prédéfinie.

8. Modèle d'une mâchoire pour la production d'une prothèse dentaire pour effectuer un procédé selon l'une des revendications précédentes, comprenant un évidement pour insérer un analogue de laboratoire (11, 21),
l'évidement étant de forme cylindrique ou conique, et un filetage d'accouplement (28) avec une butée (29) étant fourni dans l'encoche concordant avec un filetage (25) avec une butée (29) d'un analogue de laboratoire (11, 21), de sorte que l'analogue de laboratoire (11, 21) doit être disposé dans le modèle (20) d'une manière positionnement fixé, **caractérisé en ce qu'**
une position de verrouillage est fournie dans l'évidement s'enclenchant une position de verrouillage d'accouplement à l'analogue de laboratoire (11, 21) et tenant l'analogue de laboratoire (11, 21) d'une manière positionnement fixé.

9. Analogue de laboratoire pour la production d'une prothèse dentaire pour effectuer un procédé selon l'une des revendications 1 à 7, comprenant une région (13) pour insérer dans un évidement d'un modèle (20) d'une mâchoire et une pièce de tête (14) pour monter la prothèse dentaire,
la région (13) étant de forme cylindrique ou conique, et la région comprenant un filetage extérieur (25) avec une butée (29), de sorte que l'analogue de laboratoire (11, 21) doit être disposé dans un évidement ayant un filetage intérieur (28) concordant d'une manière positionnement fixé, **caractérisé en ce que**
l'analogue de laboratoire (11, 21) comprend une position de verrouillage pour s'enclencher dans une position de verrouillage d'accouplement dans l'évidement tenant l'analogue de laboratoire (11, 21) dans l'évidement d'une manière positionnement fixé.

10. Analogue de laboratoire selon la revendication 9, **caractérisé en ce que**
l'analogue de laboratoire (11, 21) a un évidement (14) dans la direction longitudinale dans la région (13).

11. Analogue de laboratoire selon l'une des revendications 9 ou 10, **caractérisé en ce que**
l'analogue de laboratoire (11, 21) comprend un moyen de fixation (23) pour fixer une structure (22) ou un pilier (22).

12. Analogue de laboratoire selon l'une des revendications 9 à 11, **caractérisé en ce que** l'analogue de laboratoire (11, 21) comprend des contre-dépouilles pour recevoir un ciment dans la région (13), de sorte que l'analogue de laboratoire (11, 21) est insérable bifonctionnellement aussi au moyen d'une technique de cimentation.
